(19) Europäisches Patentamt
European Patent Office
Office européen des brevets

(11) Publication number: **0 331 729**
**A1**

(12) # EUROPEAN PATENT APPLICATION

published in accordance with Art. 158(3) EPC

(21) Application number: **87907528.1**

(22) Date of filing: **13.11.87**

Data of the international application taken as a basis:

(86) International application number:
**PCT/JP87/00883**

(87) International publication number:
**WO88/03812 (02.06.88 88/12)**

(51) Int. Cl.$^3$: **A 61 L 29/00**
A 61 L 31/00

(30) Priority: **17.11.86 JP 271743/86**

(43) Date of publication of application:
**13.09.89 Bulletin 89/37**

(84) Designated Contracting States:
**BE DE FR GB IT NL SE**

(71) Applicant: **Terumo Kabushiki Kaisha**
**No. 44-1, Hatagaya 2-chome Shibuya-ku**
**Tokyo 151(JP)**

(72) Inventor: **NAGAI, Hiroshi**
**Terumo Kabushiki Kaisha 2656-1, Ohbuchi**
**Fuji-shi, Shizuoka 417(JP)**

(74) Representative: **Henkel, Feiler, Hänzel & Partner**
**Möhlstrasse 37**
**D-8000 München 80(DE)**

(54) **MEDICAL IMPLEMENTS.**

(57) Medical implements which are substantially constituted by a soft vinyl chloride resin composition prepared by compounding 10 to 80 parts by weight of tri-n-alkyl trimellicate per 100 parts by weight of a vinyl chloride resin. The implements show good physical properties such as gas permeability and do not release harmful plasticizers.

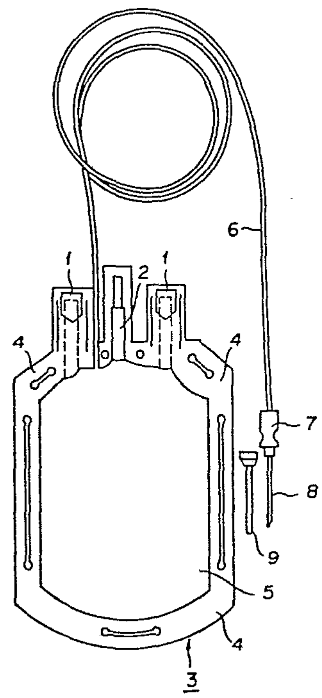


EP 0 331 729 A1

TITLE MODIFIED
see front page



DESCRIPTION

# MEDICAL DEVICE

[Technical Field]

This invention relates to a medical device. More particularly, this invention relates to a medical device which is substantially free of blood extractable plasticizer and excels in gas permeability.

[Background Art]

In the conventional blood bag, the blood platelets in the blood preserved in the bag decrease in their capacity for aggregation to about 60% of the level 6 hours of after blood collection and to about 40% at 24 hours thereof. From the standpoint of effective utilization of blood platelet preparations, there has been frequently expressed in the industry an earnest desire to develop a preserving container made of a plastic substance capable of preserving blood for a long time, namely a preserving container of a plastic substance possessing compatibility to blood, and other devices of the same nature.

As such preservnig containers or other medical devices made of a plastic substance as described above, those made of flexible vinyl chloride resin have been so far brought into extensive use on account of their desirability in fabricability, thermal stability, and cost. The flexible vinyl chloride resin generally contains, as a plasticizer, such a phthalic ester as di-2-ethylhexyl phthalate (DOP) in an amount approximately in the range of 30 to 60% by weight.

The phthalic ester, however, possesses a conspicuous migrating property. When the medical device made of the flexible vinyl chloride resin is exposed to blood, the phthalic ester is observed to migrate from the medical device and pass into the blood. There has been entertained a desire to develop a measure for solving this problem ["Ikigaku (medicomechanics)," 54, 221 (1984)] It has been known that the phthalic ester migrating from the blood bag produces an

adverse effect on the blood components, particularly platelets, preserved in the blood bag [Journal of Japan Blood Transfusion Society, 28, 282 (1982)].

For the solution of this problem, a flexible vinyl chloride resin containing 2-ethylhexyl trimellitate as a plasticizer has been proposed. The flexible vinyl chloride resin using this particular plasticizer, however, has a disadvantage in terms of cost because it is required to use the plasticizer in a large amount for the purpose of ensuring impartation of sufficient permeability of gas to the blood bag, for example.

As a plasticizer incapable of migrating in the vinyl chloride resin, a polyester type plasticizer has been known to the art. Generally this polyester type plasticizer is formed mainly of a fatty acid ester. The flexible vinyl chloride resin incorporating this polyester type plasticizer, therefore, is inferior in water-proofness, hydrolyzing property, etc. to the flexible vinyl chloride resin incorporating therein a phthalic ester or a trimellitic ester. Further it is destined to suffer from poor meability of gas.

A medical device which precludes the problem of an adverse effect on the blood components, particularly the blood platelets due to the migration of a plasticizer and, at the same time possesses highly satisfactory permeability of gas necessary for the preservation of blood remains yet to be developed.

An object of this invention, therefore, is to provide a novel medical device. Another object of this invention is to provide a medical device which is substantially free of blood extractable plasticizer and excels in perviousness to gas. Yet another object of this invention is to provide a medical device which is easy of formation and fabrication and is also easy of sterilization.

[Disclosure of Invention]

The objects described above are accomplished by a medical device characterized by being formed substantially of a flexible vinyl chloride type resin composition produced by combining 100 parts by weight of a vinyl chloride type resin and 10 to 80 parts by weight of a trimellitic tri-n-alkyl ester represented by the general formula (I):

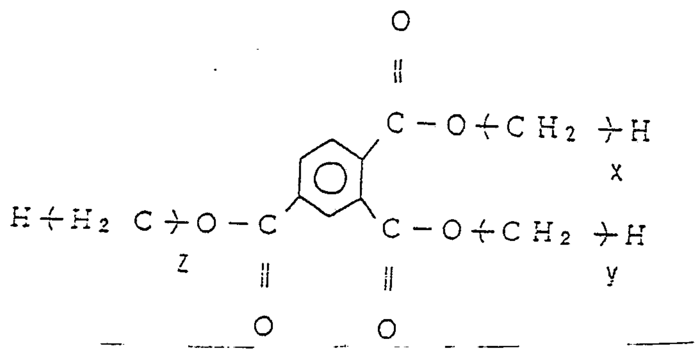

(I)

wherein x, y, and z are independently a number in the range of 4 to 14.

This invention further discloses a medical device, wherein the flexible vinyl chloride type resin composition further incorporates therein 1 to 18 parts by weight of a stabilizer, based on 100 parts by weight of the vinyl chloride type resin. This invention also discloses a medical device, wherein the symbols x, y, and z used in the general formula I independently are a number in the range of 8 to 10. Further, this invention discloses a medical device, wherein the trimellitic tri-n-alkyl ester represented by the general formula I is trimellitic tri-n-octyl ester. This invention also discloses a medical device, wherein the oxygen gas permeability coefficient is not less than 5.0 x $10^2$ml.mm/$m^2$.day.atm (30°C). This invention discloses a medical device, wherein the stabilizer is selected from among epoxidized vegetable oils and metallic soaps. This invention further discloses a medical device, which is used as exposed to blood. This invention also discloses a medical device, which is adapted to serve as a blood bag.

[Brief Description of Drawing]

The accompanying drawing is a front view illustrating a typical medical device as one preferred embodiment of the present invention.

[Best Mode for Carrying Out of the Invention]

The medical device of the present invention is characterized by being formed substantially of a flexible vinyl chloride type resin composition produced by combining 100 parts by weight of a vinyl chloride type resin and 10 to 80 parts by weight of a trimellitic tri-n-alkyl ester represented by the general formula (I). The flexible vinyl chloride type resin composition using a trimellitic tri-n-alkyl ester represented by the general formula (I) as a plasticizer sparingly admits of release of the plasticizer and exhibits at least equivalent quality in terms of perviousness to gas, fabricability, flexibility, and heatresistance as compared with the conventional flexible vinyl chloride type resin composition using a phthalic ester such DOP. The medical device which is formed of the flexible vinyl chloride type resin composition preeminently excels in quality.

Now, the present invention will be described in detail below with referene to preferred embodiments.

The medical devices which advantageously embody this invention include medical devices such as body fluid preserving containers represented by blood bag, catheters, and blood transfusion set, containers for the medical devices mentioned above, and containers for tablets and other forms of medicines, for example. Particularly since the flexible vinyl chloride type resin composition sparingly admits of release of the plasticizer and exhibits desirable perviousness to oxygen gas and, therefore, possesses highly desirable preservability for blood, particularly blood platelets, it can be used advantageously for the formation of medical devices such as blood bag which are designed to contact blood.

The vinyl chloride type resin as one of the components of the flexible vinyl chloride type resin composition of which the medical device of this invention is formed is a homopolymer of vinyl chloride or a copolymer of not less than 50% by weight, more preferably not less than

60% by weight, and most preferably not less than 65% by weight, of vinyl chloride and the balance of a monomer copolymerizable with vinyl chloride. The average dgree of polymerization of the vinyl chloride type resin is in the range of 700 to 3,000, preferably 1,000 to 2,000. The comonomers usable effectively with the vinyl chloride component include vinylidene chloride, ethylene, propylene, vinyl acetate, vinyl bromide, vinyl fluoride, styrene, vinyl toluene, vinyl pyridine, acrylic acid, alkyl acrylates (such as, for example, methyl acrylate, ethyl acrylate, isopropyl acrylate, n-butyl acrylate, and 2-ethylhexyl acrylate), methacrylic acid, alkyl methacrylates (such as, for example, methyl methacrylate, ethyl methacrylate, and 2-ethylhexyl methacrylate), acrylonitrile, and methacrylonitrile, for example. The vinyl chloride resin, when necessary, may incorporate therein styrene-acrylonitrile copolymer or styrene-methacrylonitrile copolymer.

As a plasticizer, a trimellitic tri-n-alkyl ester represented by the general formula (I) is used:

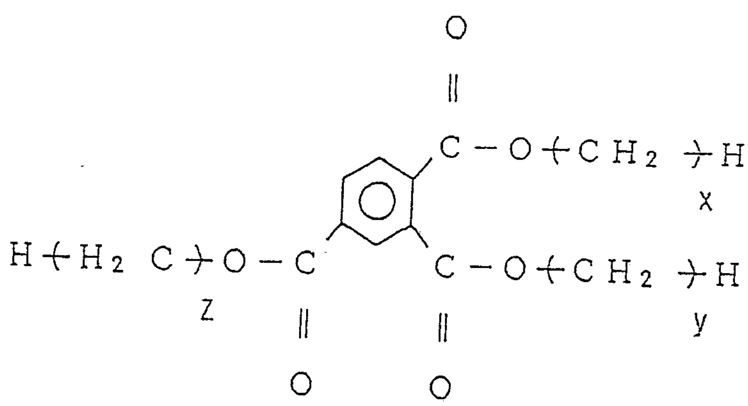

(I)

In the general formula I, x, y, and z independently are a number in the range of 4 to 14, preferably 8 to 10, providing that x, y, and z are not required to stand for one and the same number. This invention assigns the range of 4 to 14 for the number of carbon atoms alkyl chain in the trimellitic tri-n-alkyl ester because the ester used as a plsticizer in the resin composition migrates heavily in the composition when the number of carbon atoms is less than 4

and the ester is deficient in plasticizing efficiency when the number of carbom atoms exceeds 14. The alkyl chain of the ester must be a linear chain. This is because the resin composition does not acquire sufficient perviousness to gas when the alkyl chain is a branched chain. The trimellitic tri-n-alkyl esters represented by the general formula I include trimellitic tri-n-butyl ester, trimellitic tri-n-pentyl ester, trimellitic tri-n-hexyl ester, trimellitic tri-n-heptyl ester, trimellitic tri-n-octyl ester, trimellitic tri-n-nonyl ester, trimellitic tri-n-decyl ester, trimellitic tri-n-undecyl ester, trimellitic tri-n-dodecyl ester, trimellitic tri-n-tridecyl ester, trimellitic tri-n-tetradecyl ester, 1,2-di-n-octyl-4-n-nonyl trimellitate, 1,2-di-n-nonyl-4-n-decyl trimellitate, 1,2-di-n-decyl-4-n-undecyl trimellitate, and 1,2-di-n-undecyl-4-n-dodecyl trimellitate, for example. Among other trimellitic tri-n-alkyl ester mentioned above, trimellitic tri-n-octyl ester proves to be particularly desirable.

In the flexible vinyl chloride type resin composition contemplated by the present invention, the trimellitic tri-n-alkyl ester represented by the general formula I is used in an amount in the range of 10 to 80 parts by weight, preferably 40 to 60 parts by weight, based on 100 parts by weight of the vinyl chloride resin mentioned above. The reason for this range is that no sufficient softness is imparted to the composition when the amount of the trimellitic tri-n-alkyl ester represented by the general formula I to be used is less than 10 parts by weight and the flexible vinyl chloride resin composition is deficient in properties and succumbs to the phenomenon of breeding when the amount exceeds 80 parts by weight.

Besides the trimellitic tri-n-alkyl ester represented by the general formula I, the flexible vinyl chloride type resin composition of the present invention may

incorporate therein other plasticizer in an amount incapable of interfering with the accomplishment of the objects of this invention.

The flexible vinyl chloride type resin composition of the present invention, similarly to the conventional flexible vinyl chloride resin composition, may incorporate therein 0.01 to 5 parts by weight, preferably 0.05 to 3 parts by weight, of a metallic soap formed between a metal such as calcium or zinc and stearic acid, lauric acid, ricinolic acid, or naphthenic acid, and optionally 1 to 18 parts by weight, preferably 5 to 10 parts by weight, of a stabilizer such as epoxidized soybean oil or epoxidized linseed oil. It may further incorporate therein other additives such as slip additive and antioxidant.

The medical device of the present invention is obtained by forming the flexible vinyl chloride type resin composition in a stated shape. The method of adhesion resorting to the technique of high-frequency sealing employed for the conventional flexible vinyl chloride type resin composition can be similarly used for the formation of the medical device.

Now, the medical device of the present invention will be described below as embodied in a blood bag, with reference to the accompanying drawing. The drawing illustrates a blood bag connected to a blood collecting needle. A blood bag 3 made of the flexible vinyl chloride type resin composition mentioned above and provided with several outlets 1 fitted with a peel tab and a discharge outlet 2 has a peripheral part 4 thereof heat-sealed by high-frequency heating or some other suitable heating means. A tube made of the same flexible vinyl chloride type resin composition and adapted to communicate with the inner empty space of the blood bag 3 is connected to the blood bag 3. A needle base 7 is attached to the leading end of the tube 6 and a puncturing needle 8 is fitted to the needle base 7. A cap 9 is attached to the puncturing needle 8. Where the medical device is destined to contact blood like the blood

bag, the fact that it should possess an oxygen gas permeation coefficient exceeding 5.0 x $10^2$ ml.mm/$m^2$ day· atm (30°C), preferably in the range of 5.2 X $10^2$ to 8.0 x $10^2$ ml.mm/$m^2$.day. atm, constitutes itself a characteristic feature particularly desirable from the view point of blood preservation.

This invention has been described as embodied in the blood bag. The other medical devices such as body fluid preserving container, catheter, blood transfusion set, and blood circuit, containers for the medical devices mentioned above, and containers for tablets and other forms of medicines are similarly formed advantageously with the flexible vinyl chloride type resin composition containing, as a plasticizer therefor, the trimellitic tri-n-alkyl ester of the general formula I.

The medical device produced by the present invention is given a sterilizing treatment before it is put to use. This sterilizing treatment is generally carried out in an autoclave at about 121°C for about 60 minutes. The medical device of this invention has ample thermal stability enough to withstand the heat of the sterilization treatment using an autoclave.

Now, working examples will be cited below to aid further in the comprehension of this invention. It should be noted that these working examples are offered purely for the purpose of illustration of the invention and are not intended to limit the scope of the present invention in any sense.

Example 1

A resin composition prepared by mixing 100 parts by weight of polyvinyl chloride (average degree of polymerization 1,300), 52 parts by weight of trimellitic tri-n-octyl ester as a plasticizer, and 0.1 part by weight of a Ca-Zn type stabilizer and 8 parts by weight of an epoxidized soybean oil both as stabilizers was thoroughly kneaded with two rolls at 150°C and rolled in the form of sheet 0.4 mm in thickness. Miniature bags were made of the sheet, sterilized in an autoclave, and sterilely filled each

with 5 ml of human blood plasma, and then horizontally agitated (80 cycles/second) at 22°C for five days. At the end of the shaking, the blood plasma was assayed for the amount of plasticizer released from the bag into the blood plasma by high-performance liquid chromatography. Separately, test pieces cut from the aforementioned sheet were tested for oxygen gas permeation coefficient and carbon dioxide permeation coefficient with a gas permeability tester (Gas Permeability Tester L-100, Lyssy Corp.) at 30°C and further tested for 100% modulus and tensile strength at 23°C. The results were as shown in Table 1.

The extraction test was performed by the method title "Method for Testing Transfusion Quality Plastic Container" specified in the Commentary of the 10th edition of the Japanese Pharmacopoeia. The results were as shown in Table 2.

Blood bags were made of the sheet in such a manner as to be possessed of an inner surface area of $15cm^2$ and were sterilized with high-pressure steam. They were sterilely filled each with 6 ml of platelet concentrate adjusted to about 1,000,000 platelets/$mm^3$ and were preserved. Aliquots of the platelet concentrate taken before the dispensation, at 24 hours after the dispensation, and at 72 hours after the dispensation were tested for number of platelets, pH, $P_{O_2}$ , $P_{CO_2}$ , hypotonic the shock recovery ratio (%) [% HSR], and $ADP^2$ induced platelet aggregation. The results were as shown in Table 3. In the test, the number of platelets was determined with an automatic blood cell counter (ELT-8, Ortho Instrument Corp.) and the pH, $pCO_2$ and $pO_2$ were determined with a blood gas analyzer ( "BK-MK2, PHM73", Radiometer Corp.). %HSR was determined by observing the change in absorbance at a wavelength of 610 mm during the decline of the osmotic pressure from the isotonic level to 2/3 of the isotonic level of platelet-rich blood plasma adjusted to 3 x $10^5$ platelets/$mm^3$. The ability of aggregation induced by ADP was determined with an automatic platelet aggregometer (Agglicoder 3210, Kyoto Daiichi-Kagaku K.K.) using

platelet-rich blood plasma adjusted to $3 \times 10^5$ platelets/$mm^3$ and also using ADP of final concentration of $10^{-5}$ M as a aggregation-inducing substance.

Control 1

A resin composition prepared by mixing 100 parts by weight of polyvinyl chloride (average degree of polymerization 1,300), 52 parts by weight of DOP as a plasticizer, and 0.1 part by weight of a Ca-Zn type stabilizer and 8 parts by weight of an epoxidized soybean oil both as stabilizers was thoroughly kneaded with two rolls at 150°C and then rolled in the form of sheet 0.4 mm in thickness. The sheet was tested for amount of released plasticizer, physical properties, test for eluate, and time course of platelet concentrate. The results were as shown in Tables 1 to 3.

Table 1

| Item | Example 1 | Control 1 |
| --- | --- | --- |
| Amount of release into human blood plasma (μg/ml) | 0.5 | 554 |
| Oxygen gas permeability coefficient (ml.mm/$m^2$.day.atm) | $5.8 \times 10^2$ | $4.6 \times 10^2$ |
| Carbon dioxide gas permeability coefficient (ml.mm/$m^2$.day.atm) | $2.6 \times 10^3$ | $2.1 \times 10^3$ |
| 100% Modulus (kgf/$cm^2$) | 105 | 93 |
| Tensile strength (kgf/$cm^2$) | 215 | 209 |

Table 2

| Test for eluate | Example 1 | Control 1 | (Standard value) |
|---|---|---|---|
| $\Delta$ pH | 1.01 | 0.94 | 1.5 max. |
| $\Delta$ $KMnO_4$ | 0.28 | 0.75 | 1.0 max. |
| Ultraviolet absorption (220 nm) | 0.065 | 0.050 | 0.08 max. |
| " (241 nm) | 0.031 | 0.030 | 0.05 max. |

Table 3

| | Plasticizer used | Time | Number of platelets per $mm^3$ | pH | $pCO_2$ (mmHg) | $pO_2$ (mmHg) | %HSR (%) | ADP induced aggregation (%) |
|---|---|---|---|---|---|---|---|---|
| | | Before dispensation | $1.08 \times 10^6$ | 7.10 | 58 | 120 | 80 | 83 |
| Example 1 | TnOTM | 24 hours after dispensation | $1.00 \times 10^6$ | 7.37 | 19 | 138 | 60 | 32 |
| | | 72 hours after dispensation | $0.99 \times 10^6$ | 7.00 | 12 | 145 | 41 | 30 |
| Control 1 | DOP | 24 hours after dispensation | $0.98 \times 10^6$ | 7.36 | 23 | 131 | 55 | 24 |
| | | 72 hours after dispensation | $0.86 \times 10^6$ | 6.50 | 18 | 141 | 16 | 23 |

[Industrial Applicability]

As described above, this invention is directed to a medical device characterized by being formed substantially of a flexible vinyl chloride type resin composition produced by combining 100 parts by weight of a vinyl chloride type resin and 10 to 80 parts by weight of a trimellitic tri-n-alkyl ester represented by the general formula (I). The medical device, therefore, is substantially free of blood extractable plasticizer and excels in permeability of gas. In the other properties such as fabricability, softness, and thermal stability, for example, this medical device is favorably comparable with the medical device formed of the conventional flexible vinyl chloride type resin composition containing a phthalic ester as a plasticizer. Thus, it deserves to be called a preeminently excellent medical device. Particularly where the medical device of this invention is destined to contact blood like a blood bag, owing to the sparing migration of plasticizer and the high permeability to gas, the medical device exhibits a highly desirable preserving property for blood, particularly for platelets, and enables blood components to be preserved for a long time. In the medical device of this invention, when the flexible vinyl chloride type resin composition further incorporates therein 1 to 18 parts by weight of a stabilizer, preferably a stabilizer selected from among epoxidized vegetable oils and metallic soaps, or when the trimellitic tri-n-alkyl ester represented by the general formula I is such that the alkyl group thereof has 8 to 10 carbon atoms, preferably when the trimellitic tri-n-alkyl ester is trimellitic tri-octyl ester, the medical device manifests the properties mentioned above to a greater advantage. Thus, the medical device of the present invention extensively contributes to the field of blood transfusion or medicine.

WHAT IS CLAIMED IS

1. A medical device characterized by being formed substantially of a flexible vinyl chloride type resin composition produced by combining 100 parts by weight of a vinyl chloride type resin and 10 to 80 parts by weight of a trimellitic tri-n-alkyl ester represented by the general formula (I):

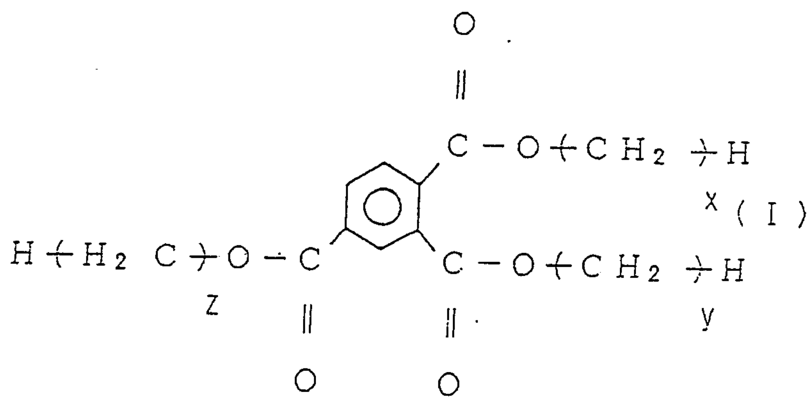

(I)

wherein x, y, and z are independently a number in the range of 4 to 14.

2. A medical device according to Claim 1, wherein said flexible vinyl chloride type resin composition further incorporates therein 1 to 18 parts by weight of a stbilizer, based on 100 parts by weight of said vinyl chloride type resin.

3. A medical device according to Claim 1 or Claim 2, wherein x, y, and z in said general formula are independently a number in the range of 8 to 10.

4. A medical device according to Claim 3, wherein said trimellitic tri-n-alkyl ester represented by the general formula I is trimellitic tri-n-octyl ester.

5. A medical device according to any one of claim s 1 to 4, wherein the oxygen gas permeability coefficient is not less than 5.0 x $10^2$ ml.mm/$m^2$.day.atm (30°C).

6. A medical device according to any one of Claims 2 to 5, wherein said stabilizer is selected from among epoxidized vegetable oils and metallic soaps.

7. A medical device according to any one of Claims 1 to 6, which is destined to contact blood.

8. A medical device according to Claim 7, wherein said medical device is a blood bag.

1/1

0331729

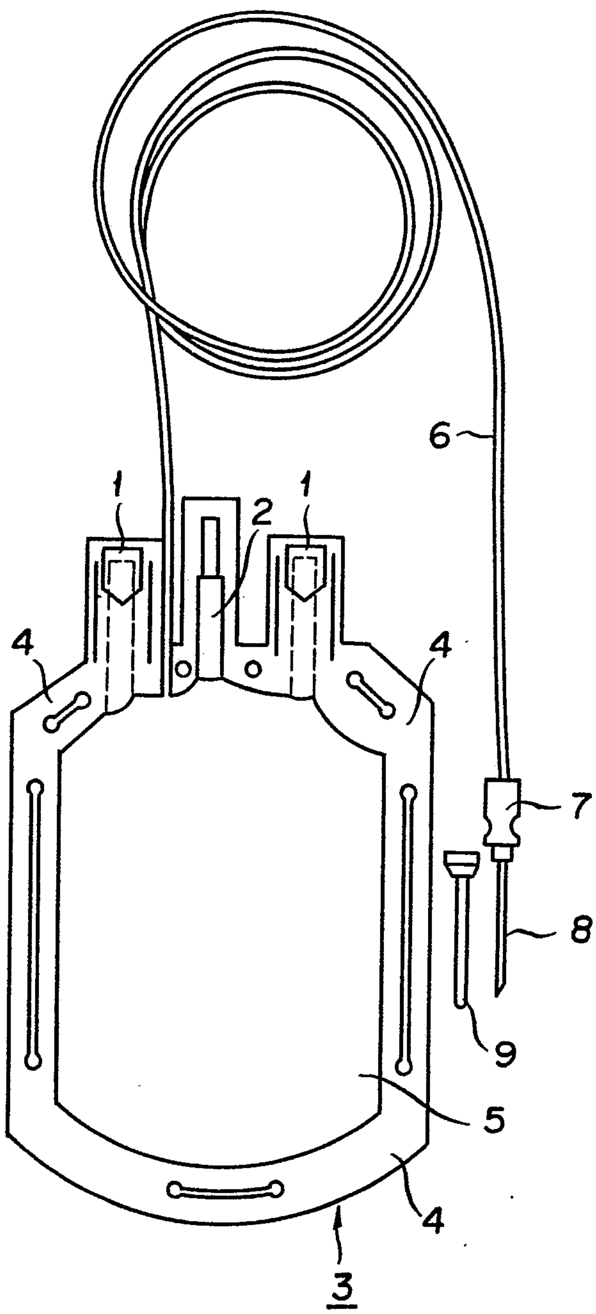

# INTERNATIONAL SEARCH REPORT

0331729

International Application No PCT/JP87/00883

**I. CLASSIFICATION OF SUBJECT MATTER** (if several classification symbols apply, indicate all) [3]

According to International Patent Classification (IPC) or to both National Classification and IPC

Int.Cl[4] A61L29/00, A61L31/00

**II. FIELDS SEARCHED**

Minimum Documentation Searched [4]

| Classification System | Classification Symbols |
|---|---|
| IPC | A61L29/00, A61L31/00 |

Documentation Searched other than Minimum Documentation to the Extent that such Documents are Included in the Fields Searched [5]

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| X | JP, A, 59-37390 (Terumo Kabushiki Kaisha) 29 February 1984 (29. 02. 84) Page 3, column 1, lines 1 to 19 (Family: none) | 1-8 |
| A | JP, A, 59-71760 (Terumo Kabushiki Kaisha) 23 April 1984 (23. 04. 84) Page 1, column 1, line 5 to page 2, column 2, line 6 & BE, A, 898017 & FR, A, 2534477 & DE, A, 3337880 | 1-8 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| January 6, 1988 (06.01.88) | January 18, 1988 (18.01.88) |
| **International Searching Authority** [1] | **Signature of Authorized Officer** [20] |
| Japanese Patent Office | |